# EUROPEAN PATENT APPLICATION

(11) **EP 1 036 792 A1**
(43) Date of publication of application: **20.09.2000**
(21) Application number: 99302050.2
(22) Date of filing: 17.03.1999
(51) Int. Cl.: C07D 311/20, C11B 9/00

(54) **Novel fragrance compound**

(71) Applicant: QUEST INTERNATIONAL B.V., 1411 GP Naarden (NL)
(72) Inventor: Munro, David, Maidstone, Kent, ME16 0HF (GB)
(74) Representative: Matthews, Heather Clare

(57) **Abstract**

2-chromanones substituted with three alkyl groups, one in the 4-position, having a total number of carbon atoms in the range 14 to 18 are novel compounds exhibiting musk odour properties, which find use in perfumes and perfumed products. The 4,6,8-trialkyl substitution pattern is particularly preferred.

## Description

### Field of the Invention

This invention concerns novel fragrance compounds, and perfumes and perfumed products comprising the novel compounds.

### Summary of the Invention

In one aspect the present invention provides a 2-chromanone substituted with three alkyl groups, R₁, R₂ and R₃, one of the alkyl groups, R₁, being in the 4-position, the chromanone having a total number of carbon atoms in the range 14 to 18.

For brevity and simplicity, such materials will be referred to as "the chromanone", "the novel chromanone" or "the chromanone of the invention".

It has been found that the novel chromanones possess musk odour properties of great interest to the fragrance industry, and can be accessed from inexpensive starting materials, unlike the macrocyclic ketones and esters whose musk properties are well known. Also, unlike many members of the polycyclic musk class, it is anticipated that the molecules of the present invention will exhibit favourable biodegradation properties, thereby preventing their accumulation in the environment.

The chromanones of particular interest are:
4,5,7-Trialkyl-2-chromanones 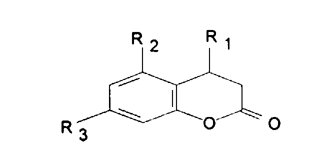 4,6,7-Trialkyl-2-chromanones 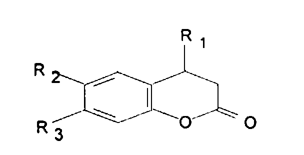 4,5,8-Trialkyl-2-chromanones 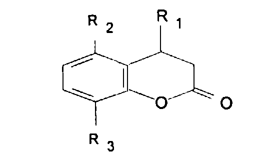 4,7,8-Trialkyl-2-chromanones 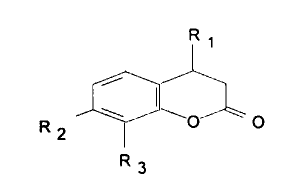 4,5,6-Trialkyl-2-chromanones 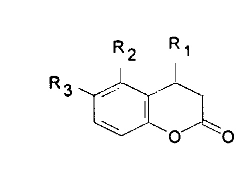 4,6,8-Trialkyl-2-chromanones. This substitution pattern is particularly preferred.

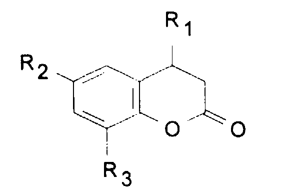

In each of these molecules, the total number of carbon atoms must be in the range of 14 to 18; therefore the alkyl groups R₁, R₂ and R₃ contribute a total of 5 to 9 carbon atoms. These groups may be linear or branched. It is particularly preferred that the group R₁ represents a linear, lower alkyl group of from 1 to 3 carbon atoms. The groups R₂ and R₃ are preferably branched, particularly isopropyl or tert-butyl groups. R₂ and R₃ may be the same or different.

The odour properties of the chromanones of the invention mean that a chromanone or mixture of chromanones in accordance with the invention may be used as such to impart, strengthen or improve the odour of a wide variety of products, or may be used as a component of a perfume (or fragrance composition) to contribute its odour character to the overall odour of such perfume. The chromanones are stable and substantive. For the purposes of this invention a perfume is intended to mean a mixture of fragrance materials, if desired mixed with or dissolved in a suitable solvent or mixed with a solid substrate, which is used to impart a desired odour to the skin and/or product for which an agreeable odour is indispensable or desirable. Examples of such products are: fabric washing powders, washing liquids, fabric softeners and other fabric care products; detergents and household cleaning, scouring and disinfection products; air fresheners, room sprays and pomanders; soaps, bath and shower gels, shampoos, hair conditioners and other personal cleansing products; cosmetics such as creams, ointments, toilet waters, preshave, aftershave, skin and other lotions, talcum powders, body deodorants and antiperspirants, etc.

Other fragrance materials which can be advantageously combined with one or more chromanones according to the invention in a perfume are, for example, natural products such as extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carbocyclic, and heterocyclic compounds.

Such fragrance materials are mentioned , for example, in S. Arctander, Perfume and Flavor Chemicals (Montclair, N.J., 1969), in S. Arctander, Perfume and Flavor Materials of Natural Origin (Elizabeth. N.J., 1960) and in "Flavor and Fragrance Materials - 1991", Allured Publishing Co. Wheaton, III. USA.

Examples of fragrance materials which can be used in combination with one or more chromanones according to the invention are: geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nonpol, nopyl acetate, 2-phenyl-ethanol, 2-penylethyl acetate, benzyl alcohol, benzyl acetate, benzyl salicylate, styrallyl acetate, benzyl benzoate, amyl salicylate, dimethylbenzyl-carbinol, trichloromethylphenyl-carbinyl acetate, p-tert-butylcyclohexyl acetate, isononyl acetate, vetiveryl acetate, vetiverol, α-hexylcinnamaldehyde, 2-methyl-3-(p-tert-butylpheyl)propanal, 2-methyl-3-(p-isopropylphenyl)propanal, 2-(p-tert-butylpheyl)-propanal, 2,4-dimethyl-cyclohex-3-enyl-carboxaldehyde, tricyclodecenyl acetate, tricyclodecenyl propionate,4-(4-hydroxy-4-methylpentyl)-3-cyclohexenecarboxyaldehyde, 4-(4-methyl-3-pentenyl)-3-cyclohexenecarboxaldehyde, 4-acetoxy-3-pentyl-tetrahydropyran, 3-carboxymethyl-2-pentylcyclopentane, 2-n-heptylcyclopentanone, 3-methyl-2-pentyl-2-cyclopentenone, n-decanal, n-dodecanal, 9-decenol-1, phenoxyethyl isobutyrate, phenyl-acetaldehyde dimethyl-acetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks, tetralin musks, isochroman musks, macrocyclic ketones, macrolactone musks, ethylene brassylate.

Solvents which can be used for perfumes which contain a chromanone according to the invention are, for example: ethanol, isopropanol, diethyleneglycol monoethyl ether, dipropylene glycol, diethyl phthalate, triethyl citrate, isopropyl myristate, etc.

The quantities in which one or more chromanones according to the invention can be used in perfumes or in products to be perfumed may vary within wide limits and depend, inter alia, on the nature of the product, on the nature and the quantity of the other components of the perfume in which the chromanone is used and on the olfactive effect desired. It is therefore only possible to specify wide limits, which, however, provide sufficient information for the specialist in the art to be able to use a chromanone according to the invention for his specific purpose. In perfumes an amount of 0.01% by weight or more of a chromanone according to the invention will generally have a clearly perceptible olfactive effect. Preferably the amount is 0.1 to 80% by weight, more preferably at least 1%. The amount of the chromanone according to the invention present in products will generally be at least 10 ppm by weight, preferably at least 100 ppm, more preferably at least 1000 ppm. However, levels of up to about 20% by weight may be used in particular cases, depending on the product to be perfumed.

In a further aspect the invention provides a perfume comprising one or more chromanones of the invention in an olfactively effective amount.

The invention also covers a perfumed product comprising one or more chromanones of the invention.

The invention will be further described, by way of illustration, in the following Examples.

### Example 1. Synthesis of 6,8-Diisopropyl-4-methyl-2-chromanone

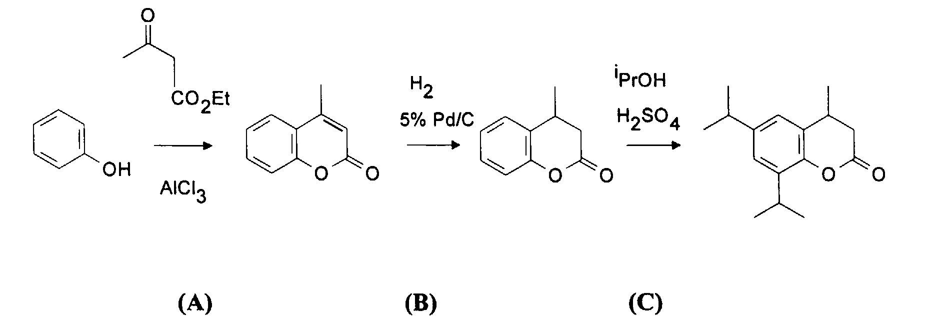

### (A) Synthesis of 4-Methyl-2H-2-chromenone

Phenol (100g : 1.06mol) and ethyl acetoacetate (160g : 1.23mol) were dissolved in nitrobenzene (150ml), heated to 100°C, and a solution of AlCl₃ (282g : 2.12mol) in nitrobenzene (1100ml) was added, with stirring, over 30-40 minutes. The temperature was then increased to 130°C and maintained at this temperature for 3 hours. After cooling to ambient temperature, the reaction mixture was decomposed by dropwise addition of a mixture of concentrated hydrochloric acid (140ml) in water (140ml). The reaction mixture was then steam distilled, taking off 200mls of distillate, which was discarded.

The residue was hot filtered through Celite (Celite is a Trade Mark), and the organic layer was vacuum distilled. Celite is a diatomaceous earth, about 95% SiO₂. After recovery of the nitrobenzene (bp 60°C @ 1mm Hg), the product was obtained as a dark yellow oil (bp 182°C @ 1 mm Hg), which solidified almost immediately. Flash chromatography on silica, using a 50/50 mixture of hexane and diethyl ether as the eluent, gave 4-methyl-2*H*-2-chromenone as very pale yellow crystals (105g : 62%), which could be recrystallised, mp 85°C (ethyl acetate/hexane).

### (B) Synthesis of 4-Methylchromanone

4-Methyl-2*H*-2-chromenone (100g : 0.62mol) was dissolved in ethanol (500ml), and 5% Pd/C (10g) added. The reaction mixture was added to a 1L Buchi autoclave, and hydrogenated at 6bar hydrogen pressure, while applying external heating. There was no hydrogen uptake until the temperature of the reaction medium reached 80-90°C, whereupon hydrogen uptake was rapid, and complete within approximately 90 minutes. The reaction mixture was allowed to cool before removing the catalyst by filtration through Celite.

TLC [silica : hexane 70%, ethyl acetate 30%] showed a single, less polar product, GLC [SE54 : 50-250° @ 12°Cmin⁻¹] indicated a purity of 95.6%.
The ethanol was removed *in vacuo* and the residue was vacuum distilled to give 4-methylchromanone (85.1 g : 84%), bp 96°C @ 0.3 mm Hg, M+ 162.

GLC[SE 54 : 50-250°C @ 12°Cmin⁻¹ ] now indicated a purity of 97.8%.
¹³C NMR (CDCl₃) : 20.0888(CH₃), 29.6772(CH), 36.9794(CH₂), 117.0154(CH aromatic), 124.7968(CH aromatic), 126.7313(CH aromatic), 128.0694(quaternary aromatic), 128.4518(CH aromatic), 151.4364(quaternary aromatic), 168.4876(C=O).

### (C) Synthesis of 6,8-Diisopropyl-4-methyl-2-chromanone

4-Methyl-2-chromanone (25g : 0.15mol) was dissolved in isopropyl alcohol (20g : 0.3mol), and added dropwise to concentrated H₂SO₄ (100ml), maintained at 15°C. After complete addition, the reaction mixture was allowed to stir at ambient temperature, and was sampled at intervals by quenching small aliquots in a mixture of water and diethyl ether, analysing the ether layer by GLC [SE54 : 50-250°C @ 12°Cmin⁻¹]. Representative samples were also submitted for mass spectrometry, giving the parent ions of the major products. After 72 hours, the composition of the reaction mixture was as follows :

| Retention time / minutes | Percentage | Parent ion |
|---|---|---|
| 6.197 | 3.4% | M+ 204 |
| 7.266 | 65.3% | M+ 246 |
| 7.677 | 3.9% | not analysed |
| 7.994 | 24.5% | M+ 246 |
| 8.198 | 1.1% | M+ 288 |

The reaction mixture was then poured into ice (500ml), and extracted into dichloromethane. The organic layer was separated, washed, and dried over MgSO₄. TLC [silica : ethyl acetate 30%, hexane 70%] indicated two products which were both less polar than the starting material, the more polar of these being the mono-isopropyl derivative, 6-isopropyl-4-methyl-2-chromanone [M+ 204]. As this was likely to co-distill with the desired product, it was removed by chromatography (as TLC), leaving a colourless oil (30.1 g).
GLC [SE54 : 100-250°C @ 12°Cmin⁻¹] showed the following composition :

| Retention time / minutes | Percentage |
|---|---|
| 7.303 | 71.3% |
| 7.585 | 3.1% |
| 8.020 | 24.3% |

This material was now fractionated *in vacuo,* using a 12inch column, packed with metal foil. This gave 6,8-diisopropyl-4-methyl-2-chromanone as a colourless oil (6.9g), bp 105°C @ 0.3mm Hg.
GLC [SE54 : 100-250°C @ 12°Cmin⁻¹ ] showed the following composition :

| Retention time / minutes | Percentage |
|---|---|
| 7.296 | 93.4% |
| 7.679 | 1.2% |
| 7.985 | 4.8% |

¹³C NMR (CDCl₃) : 20.2264(CH₃), 22.9791(CH₃), 23.0709(CH₃), 24.2943(CH₃), 24.3554(CH₃), 26.9934(CH), 30.1666(CH), 34.0280(CH), 37.0177(CH₂), 121.7842(CH aromatic), 123.2523(CH aromatic), 127.6565(quaternary aromatic), 136.3809(quaternary aromatic), 145.0749(quaternary aromatic), 146.6652(quaternary aromatic), 168.8699(C=O).

The distillation residue (20.1 g) had the following composition, from which a further quantity of 6,8-diisopropyl-4- methyl-2-chromanone could be obtained, if desired :

| Retention time / minutes | Percentage |
|---|---|
| 7.291 | 52.5% |
| 7.685 | 4.5% |
| 8.033 | 41.0% |
| 8.204 | 1.1% |

In order to obtain alternative substitution patterns in these molecules, it is possible to utilise alkyl-substituted phenols as starting materials. This is demonstrated below for the synthesis of 6-(*tert*-butyl)-8-isopropyl-4-methyl-2-chromanone.

### Example 2. Synthesis of 6-(tert-Butyl)-8-isopropyl-4-methyl-2-chromanone

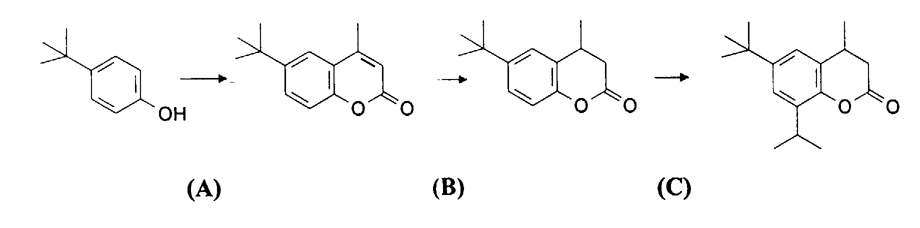

### (A) Synthesis of 6-(tert-Butyl)-4-methyl-2H-2-chromenone

4-*tert*-Butylphenol (100g : 0.67mol) and ethyl acetoacetate (126g : 0.97mol) were dissolved in nitrobenzene (100ml), heated to 100°C, and a solution of AlCl₃ (185g : 1.39mol) in nitrobenzene (600ml) was added, with stirring, over 35 minutes. After subsequently increasing the temperature to 130°C, these conditions were maintained for 3 hours, before cooling to ambient temperature, and decomposing the reaction mixture by dropwise addition of a mixture of concentrated hydrochloric acid (80ml) in water (80ml).

The reaction mixture was then steam distilled, taking off 100mls of distillate, which was discarded. The residue was hot filtered through Celite, and the organic layer was distilled *in vacuo.* After recovery of the nitrobenzene (bp 60° @ 1mm Hg), two fractions were obtained, being :
unreacted 4-*tert*-butylphenol (6.8g), bp 120°C @ 2mm Hg, and
6-(*tert*-butyl)-4-methyl-2*H*-2-chromenone (94.3g), bp 154° @ 1mm Hg.
The latter material was further purified by flash chromatography [silica : ethyl acetate 20%, hexane 80%] to give a colourless solid (89.7g). Recrystallisation gave colourless crystals, mp 119°C (hexane/ethyl acetate)

### (B) Synthesis of 6-(tert-Butyl)-4-methyl-2-chromanone

6-(*tert*-Butyl)-4-methyl-2*H*-2-chromenone (80g : 0.37mol) was dissolved in ethanol (400ml), and 5% Pd/C (8g) added. This was hydrogenated in a 1L Buchi autoclave at 6 bar hydrogen pressure, while applying external heating. There was no hydrogen uptake until the temperature of the reaction medium reached 80-90°C, whereupon hydrogen uptake was rapid, and complete within approximately 90 minutes. The reaction mixture was allowed to cool before removing the catalyst by filtration through Celite. TLC [silica : hexane 80%, ethyl acetate 20%] showed a single, less polar product. The ethanol was removed *in vacuo* to give a colourless solid, which was flash-chromatographed (as TLC) to give colourless crystals (67.9g). Following recrystallisation from hexane, these had mp 94°C.

### (C) Synthesis of 6-(tert-Butyl)-8-isopropyl-4-methyl-2-chromanone

6-(*tert*-Butyl)-4-methyl-2-chromanone (10.0g : 0.046mol) was dissolved in the minimum amount of warm isopropyl alcohol (30ml), and this solution was added dropwise, with stirring, to concentrated H₂SO₄, maintaining the temperature below 15°C. After complete addition, the reaction mixture was allowed to reach ambient temperature, and aliquots were taken and quenched into a mixture of water and diethyl ether. The organic phase of these samples was analysed by GLC, monitoring for the appearance of a new product having a retention time of approximately 7.5 minutes.

| Reaction Time | | Reaction Time | | Reaction Time | | Reaction Time | |
|---|---|---|---|---|---|---|---|
| 1 hour | | 3 hour | | 24 hour | | 48 hour | |
| Retention Time | | Retention Time | | Retention Time | | Retention Time | |
| 7.550 min | 7.5% | 7.548 min | 9.4% | 7.551 min | 19.9% | 7.550 min | 21.2% |

As there was little difference between the one day and two day result, the reaction was worked up at this stage, by pouring into a 50/50 mixture of ice water and diethyl ether (800ml). The organic layer was separated, washed, and dried over MgSO₄. TLC [silica : hexane 90%, Et₂O 10%] indicated a slightly less polar product. Chromatography (as TLC) gave the title compound as a colourless oil (1.7g), followed by recovered starting material (5.4g).
The product was short-path distilled, approx. bp 110°C @ 1mm Hg.
GLC [SE54 : 100-250°C @ 12°Cmin⁻¹]

| Retention Time / min | Percentage | Parent ion |
|---|---|---|
| 7.429 | 2.0% | M+ 260 |
| 7.586 | 91.8% | M+ 260 |
| 8.028 | 1.1% | M+ 260 |

¹³C NMR (CDCl₃) : 20.2825(CH₃), 22.9281(CH₃ of isopropyl), 23.0199(CH₃ of isopropyl), 27.0647(CH), 30.2579(CH), 31.5287(CH₃ of *t*-butyl), 34.6651(quaternary of *t*-butyl), 36.9666(CH₂), 120.8692(CH aromatic), 122.2833(CH aromatic), 127.0932(quaternary aromatic), 135.8788(quaternary aromatic), 146.3160(quaternary aromatic), 147.2641(quaternary aromatic), 168.8648(C=O).

When odour assessed by a panel of trained perfumers, both 6,8-diisopropyl-4-methyl-2-chromanone and 6-(*tert*-butyl)-8-isopropyl-4-methyl-2-chromanone were described as strong musks. In addition, the former molecule had woody and fruity nuances.

Other substituted 2-chromanones can be made in analogous manner, using the approach of Example 1 or Example 2.

## Claims

1. A 2-chromanone substituted with three alkyl groups, R₁, R₂ and R₃, one of the alkyl groups, R₁, being in the 4-position, the chromanone having a total number of carbon atoms in the range 14 to 18.

2. A chromanone according to claim 1, comprising a 4,5,7-trialkyl-2 chromanone, a 4,6,7-trialkyl-2-chromanone, a 4,5,8-trialkyl-2-chromanone, a 4,7,8-trialkyl-2-chromanone, a 4,5,6-trialkyl-2-chromanone or a 4,6,8-trialkyl-2-chromaonone.

3. A chromanone according to claim 1 or 2, wherein R₁ is a linear, lower alkyl group having from 1 to 3 carbon atoms.

4. A chromanone according to claim 3, wherein R₁ is a methyl group.

5. A chromanone according to any one of the preceding claims, wherein R₂ and R₃ is each a branched alkyl group.

6. A chromanone according to claim 5, wherein R₂ and R₃ each comprises an isopropyl group or a tert-butyl group.

7. A perfume comprising one or more chromanones in accordance with any one of the preceding claims in an olfactively effective amount.

8. A perfume according to claim 7, wherein the chromanone is present in an amount of at least 0.01% by weight.

9. A perfume according to claim 8, wherein the chromanone is present in an amount in the range 0.1 to 80% by weight.

10. A perfumed product comprising one or more chromanones according to any one of claims 1 to 6 or a perfume according to claim 7, 8 or 9.
